# EUROPEAN PATENT SPECIFICATION

(11) **EP 3 632 495 B1**
(45) Date of publication and mention of the grant of the patent: **26.05.2021**
(21) Application number: 18198416.2
(22) Date of filing: 03.10.2018
(51) Int. Cl.: A61M 27/00

(54) **AN ELECTRONIC TOOLSET FOR USE WITH MULTIPLE GENERATIONS OF IMPLANTABLE PROGRAMMABLE VALVES WITH OR WITHOUT ORIENTATION FUNCTIONALITY BASED ON A FIXED REFERENCE MAGNET**
ELEKTRONISCHER WERKZEUGSATZ ZUR VERWENDUNG MIT MEHREREN GENERATIONEN VON IMPLANTIERBAREN PROGRAMMIERBAREN VENTILEN MIT ODER OHNE AUSRICHTUNGSFUNKTIONALITÄT BASIEREND AUF EINEM FESTEN REFERENZMAGNETEN
BOÎTE À OUTILS ÉLECTRONIQUE DESTINÉE À ÊTRE UTILISÉE AVEC PLUSIEURS GÉNÉRATIONS DE SOUPAPES PROGRAMMABLES IMPLANTABLES AVEC OU SANS FONCTIONNALITÉ D'ORIENTATION BASÉE SUR UN AIMANT DE RÉFÉRENCE FIXE

(43) Date of publication of application: 08.04.2020
(73) Proprietor: Integra LifeSciences Switzerland Sàrl, 2400 Le Locle (CH)
(72) Inventor: BODEN, JR., Thomas, Plainsboro, NJ 08536 (US); D'AOUST, Patricia, Plainsboro, NJ 08536 (US); ARAZAWA, Alexander, Plainsboro, NJ 08536 (US)
(74) Representative: Small, Gary James

(56) References cited:
- EP-A2- 2 777 751
- US-A1- 2014 261 793
- US-A1- 2018 126 147
- US-A1- 2018 184 943

## Description

### BACKGROUND OF THE INVENTION

### Field of the Invention

The present invention relates to a system and method for an implantable drainage valve for drainage of a bodily fluid (e.g., cerebrospinal fluid). In particular, the present inventive system and method is directed to an electronic toolset for indicating and adjusting with automatic switching between multiple generations of implantable bodily fluid drainage valves with and without orientation functionality.

### Description of Related Art

Hydrocephalus is the accumulation of cerebrospinal fluid in the brain, resulting from increased production, or more commonly, pathway obstruction or decreased absorption of the fluid. Cerebrospinal fluid (CSF) shunts have been used for decades for the treatment of hydrocephalus. A CSF shunt involves establishing an accessory pathway for the movement of CSF to bypass an obstruction of the natural pathways.

The shunt is positioned to enable the CSF to be drained from the cerebral ventricles or sub-arachnoid spaces into another absorption site (e.g., the right atrium of the heart or the peritoneal cavity) through a system of small catheters. A regulatory device, such as a valve, may be inserted into the pathway of the catheters. In general, the valve keeps the CSF flowing away from the brain and moderates the pressure or flow rate. The drainage system using catheters and valves enables the excess CSF within the brain to be evacuated and, thereby, the pressure within the cranium to be reduced.

Some implantable valves are fixed pressure valves (i.e., monopressure valves) while others have adjustable or programmable settings. Programmable or adjustable implantable valves are desirable in that the valve pressure setting may be varied non-invasively via an external control device over the course of treatment without requiring explantation. One such conventional adjustable or programmable implantable valve using magnets is the CODMAN® HAKIM® Programmable Valve (CHPV), as disclosed in US Patent No. 4,595,390, which is assigned to DePuy Orthopedics, a J&J company related to that of the present assignee. Another programmable implantable drainage valve is the CODMAN® CERTAS® or CERTAS® Plus Programmable Valve, as disclosed in US Patent No. 8,322,365, also assigned to DePuy Orthopedics, a J&J company related to that of the present assignee. Medtronic also has a programmable implantable shunt valve Strata® controlled using magnets. The pressure setting in these aforementioned conventional programmable implantable valves may be non-invasively adjusted post implantation in the body using a rotating construct or rotor with a pair of magnets.

US2018/0126147 A1 describes an externally programmable valve assembly and controller using a magnetic field.

US2018/0184943 A1 describes a system and method of detection of spatial location and rotation of a programmable implantable valve using a magnetic field.

Each programmable implantable valve is controlled using an associated external toolset comprising one or more devices used to locate the valve, read the current valve setting and adjust the valve setting. With each improved generation or version of the programmable implantable valve an associated toolset may be required. However, often the electronic toolset used for indicating and adjusting the latest generation of the programmable implantable valve is inoperable on an earlier generation or version of the programmable implantable valve. As a result, medical personnel are required to store multiple versions of the electronic toolset (i.e., not just the latest improved version, but previous versions also) as well as properly use each electronic toolset only with the applicable programmable implantable valve. Furthermore, it is not always clear from the patient records or x-ray identification, what generation of valve is implanted in the patient based on which the appropriate toolset is selected.

It is therefore desirable to develop a universal or interchangeable electronic toolset for use in indicating and adjusting multiple generations or versions of programmable implantable valves that can automatically switch without additional input from the clinician between generations of valves with or without orientation functionality.

### Summary of the Invention

The invention is defined in appended claims 1-8. An aspect of the present invention is directed to a universal or interchangeable electronic toolset for use in indicating and adjusting multiple generations or versions of programmable implantable valves that can automatically switch without additional input from the clinician between generations of valves with or without orientation functionality.

Another aspect of the present invention relates to a method for using a universal electronic toolset for indicating and adjusting of an implantable programmable bodily fluid drainage valve whether the implantable programmable bodily fluid drainage valve includes a fixed reference magnet used to determine an angle of orientation of the implantable programmable bodily fluid drainage valve or not, wherein the implantable programmable bodily fluid drainage valve includes an adjustable valve unit having a pair of primary magnetic elements. Using a magnetic field detection sensor array in an indicator tool of the electronic toolset it is determined whether the fixed reference magnet is present in the implantable programmable bodily fluid drainage valve. If the presence of the fixed reference magnet in the implantable bodily fluid drainage valve is detected then: a center of the adjustable valve unit is located using the indicator tool of the electronic toolset; a direction of flow of the adjustable valve unit is ascertained based exclusively on electronic feedback provided by the indicator tool of the electronic toolset, without requiring manual physical palpation; the indicator tool of the electronic toolset is aligned with the located center and the direction of flow of the adjustable valve unit; and a current valve setting is read using the indicator tool of the electronic toolset. Otherwise, if the presence of the fixed reference magnet is in the implantable programmable bodily fluid drainage valve is not detected then: the center of the adjustable valve unit is located using the indicator tool of the electronic toolset; the direction of flow of the adjustable valve unit is established exclusively by manual physical palpation, without electronic feedback from the indicator tool of the electronic toolset; the indicator tool of the electronic toolset is aligned with the located center and the direction of flow of the adjustable valve unit; and the current valve setting is read using the indicator tool of the electronic toolset.

Yet another aspect of the present invention is directed to an implantable programmable bodily fluid drainage valve system including an implantable programmable bodily fluid drainage valve having an adjustable valve unit including a pair of primary magnetic elements for programming the implantable programmable bodily fluid drainage valve to a desired valve setting. The system also includes a universal electronic toolset for indicating and adjusting the implantable programmable bodily fluid drainage valve. In that, the universal electronic toolset includes an indicator tool having a magnetic field detection sensor array for: (i) detecting the pair of primary magnetic elements; and (ii) determining the presence or absence of a fixed reference magnet in the implantable programmable bodily fluid drainage valve. Circuitry is provided for determining a center of the adjustable valve unit based on the detected pair of primary magnetic elements. Such circuitry determines exclusively by electronic feedback from the toolset an angular orientation of the implantable programmable bodily fluid drainage valve, when the fixed reference magnet is present; whereas the circuitry generates on a display of the implantable programmable bodily fluid drainage valve steps for determining exclusively by manual physical palpation the angular orientation and/or center of the implantable programmable bodily fluid drainage valve, when the fixed reference magnet is absent.

### Brief Description of the Drawing

The foregoing and other features of the present invention will be more readily apparent from the following detailed description and drawings of illustrative of the invention wherein like reference numbers refer to similar elements throughout the several views and in which:
Figure 1 is a schematic perspective exploded view of a programmable implantable valve device having a fixed reference magnet in addition to the rotational primary magnets associated with the adjustable valve unit;
Figure 2 is an exploded perspective view of the adjustable valve unit of Figure 1;
Figure 3 is a top view of the adjustable valve unit of Figure 2;
Figure 4 is a side cross-sectional view of the adjustable valve unit of Figure 3 along lines 4-4;
Figure 4A is a side view of a single rotor tooth in engagement with a single lock stop;
Figure 5 is a cross-sectional view of the adjustable valve unit of figure 3 along lines 5-5;
Figure 6 is a partial cross-sectional view of the adjustable valve unit of Figure 4 approximately along lines 6-6 at a first pressure setting;
Figure 6A is a deeper cross-sectional view of the adjustable valve unit of Figure 4 approximately along lines 6A-6A at a first pressure setting;
Figures 6B-6H are partial cross-sectional view of the adjustable valve unit of Figure 4 at different, successive pressure settings;
Figure 6I is a partial cross-sectional view of the adjustable valve unit of Figure 4 at an exemplary first pressure setting illustrating the arrow marking on the programmable valve device denoting a direction of fluid flow therethrough and the fixed reference magnet;
Figure 6J is a top view of the programmable valve device of Figure 1 wherein the adjustable valve unit is at the same first pressure setting illustrated in Figure 6I and also showing the direction of flow arrow marking and positioning of the fixed reference magnet;
Figure 7 is a deeper cross-sectional view of the adjustable valve unit of Figure 4 along lines 7-7;
Figure 8 is a cross-sectional view of the adjustable valve unit of Figure 7 showing the transition to a different pressure setting;
Figure 9 is a perspective view of the spring arm unit with optional torsion spring;
Figure 9A is a top plan view of the element of Figure 9;
Figure 10 is a side cross-sectional view of the adjustable valve unit of Figure 8 along lines 10-10 showing axial lifting of the rotatable construct;
Figure 11 is a shallower partial top cross-sectional view of the adjustable valve unit of Figure 6H showing the "virtual off' position in an unconstrained condition;
Figure 12 is a side view along lines 12-12 of Figure 11;
Figure 13 is a side cross-sectional view along lines 13-13 of Figure 11;
Figure 13A is a partial cross-sectional view along lines 13A-13A of Figure 13;
Figure 14 is a perspective view of a tool set including an integrated locator/indicator tool, an adjustment tool and a screwdriver;
Figure 14A is a top perspective view of the integrated locator/indicator tool and adjustment tool of Figure 14, prior to the adjustment tool being inserted into the integrated locator/indicator tool;
Figure 14B is a top perspective view of the integrated locator/indicator tool and adjustment tool of Figure 14, with the adjustment tool inserted into a complementary cavity in the integrated locator/indicator tool;
Figure 15 is an exploded perspective view of the integrated locator/indicator tool of Figure 14;
Figure 16 is an exploded perspective view of the adjustment tool of Figure 14;
Figure 16A is a perspective view of the placement of the half round magnets on either side of the magnet shield comprising part of the magnet assembly of Figure 16;
Figure 16B is a perspective view of the assembled magnet assembly of Figure 16;
Figure 16C is a top view of the assembled bottom and middle housing sections of the adjustment tool of Figure 16 showing the internal vertical ribs;
Figure 16D is a perspective view of the assembled adjustment tool of Figure 14 without the outer housing section to illustrate the magnet assembly;

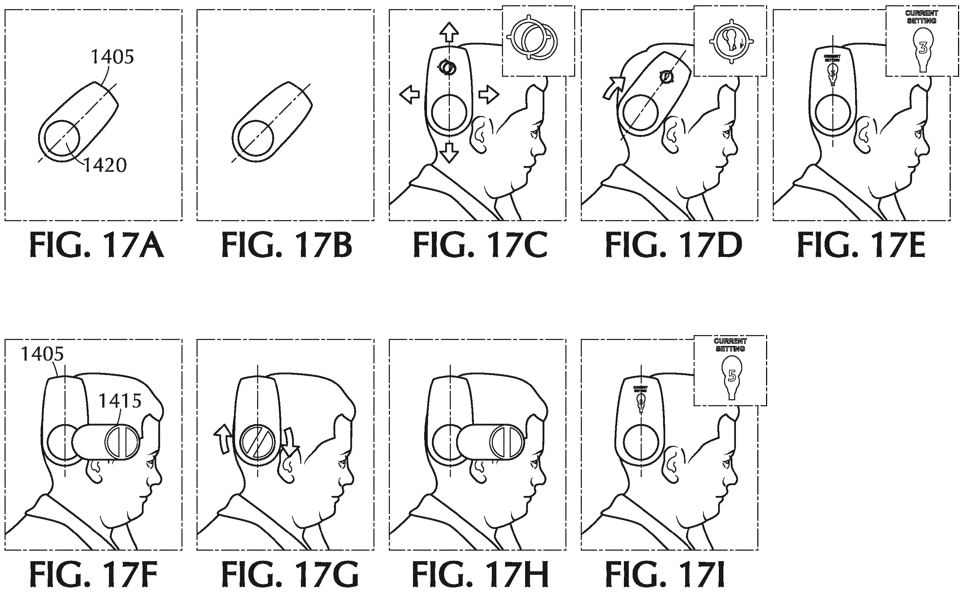

Figures 17A-17I are sequential illustrations of the steps for operating the electronic toolset in accordance with the present invention; and
Figure 18 is a flow chart of the method for ascertain whether the implanted valve is a current valve with orientation functionality (i.e., employs a fixed reference magnet) in accordance with the present invention or a previous version of the implanted valve without orientation functionality (i.e., does not employ a fixed reference magnet).

### Detailed Description of the Invention

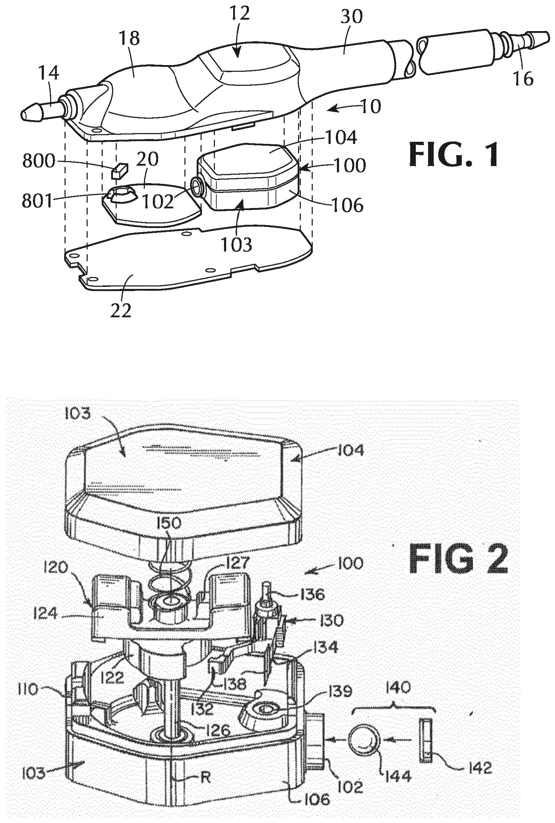

Figure 1 illustrates a programmable shunt valve device 10 having a shunt housing 12, preferably formed of a translucent material such as silicone, with proximal connector 14 and distal connector 16. A ventricular catheter or other proximal catheter is connectable to connector 14 to bring fluid into shunt housing 12. Fluid passes into sampling or pumping chamber 18 and then through a valve mechanism in inlet 102 into adjustable valve unit 100, which is shown and described in more detail below in relation to Figures 2-13A. Adjustable valve unit 100, Figure 1, includes a casing 103 formed as upper casing 104 and lower casing 106 which are joined by sonic welding in this construction. A needle guard 20, preferably formed of a rigid polymeric material, and lower casing 106 are secured within housing 12 by a backing plate 22, preferably formed of silicone reinforced with a polymeric mesh, which is bonded to housing 12 by a medical grade epoxy. A fixed reference magnet 800, as described in detail further below, is preferably seated in a bump or projection 801 on the needle guard 20.

When fluid pressure at inlet 102 exceeds a selected pressure setting within adjustable valve unit 100, fluid is admitted past a valve mechanism and then flows through valve unit outlet 110 into passage 30 of housing 12. Ultimately, fluid exits from housing 12 through distal connector 16 into a peritoneal catheter or other distal catheter.

Adjustable valve unit 100, Figure 2, includes a rotor 120, spring arm unit 130, valve mechanism 140, and a rotor retention spring 150. Rotor 120, also referred to as a rotating construct, is formed of a lower cam structure 122 having a plurality of radially flat cam surfaces, as shown and described in more detail below, and an upper, magnet housing 124 carrying magnetic elements 123 and 125, N and S pole magnets, respectively. Housing 124 also defines a finger 127 which engages a stop in upper casing 104 when rotor 120 is moved to an unconstrained condition as described below. Rotor 120 rotates about axle 126 which defines a substantially fixed axis of rotation R at a first location in casing 103.

Preferably, rotor 120 is also capable of moving along the axis of rotation, in a translational motion, to an unconstrained condition when an adjustment tool from an electronic toolset is applied to it, as described in more detail below. Retention spring 150 biases rotor 120 to a downward, normally constrained condition. Preferably, spring 150 is a coil spring having sufficient bias to resist the effect of gravity, regardless of the position of the adjustable valve unit 100, and to resist magnetic or ferrous objects, such as magnets in an integrated locator/indicator tool from the electronic toolset, as described in more detail below. However, spring 150 is insufficient to resist the effects of the adjustment tool, also described below. Lower cam section 122 has a sufficient height to ensure that cam follower 132 remains in contact with a cam surface in both the constrained and unconstrained conditions.

Spring arm unit 130 includes cam follower 132, a resilient spring element 134 as well as upper and lower axles 136, 138 at a second location in casing 103. Axle 138 turns about a bearing 139 formed of a relatively low-friction, relatively hard material such as synthetic ruby. It is desirable for casing 103, rotor 120 and spring arm unit 130 to be formed of polyethersulfone, while all spring components are formed of medical grade non-ferromagnetic stainless steel.

Valve mechanism 140 includes seat 142 and movable valve member 144. Preferably, seat 142 and valve member 144, such as a ball, are formed of the same non-ferromagnetic material such as synthetic ruby. In other constructions, the movable valve member 144 may be a disc, a cone, or other type of plug. A spherical ball is currently preferred as the moveable valve member because that shape enables tight, precise tolerances, assembly and control relative to the valve seat. Also, the position of the seat within a port can be adjusted during assembly of the valve unit to alter the actual performance value achieved at each setting, using a force versus displacement relationship. First, a mandrel checks the position of the ball, and the seat is inserted to an estimated desirable location within the port. Ball displacement is tested at one or more settings to confirm that desired performance will be achieved.

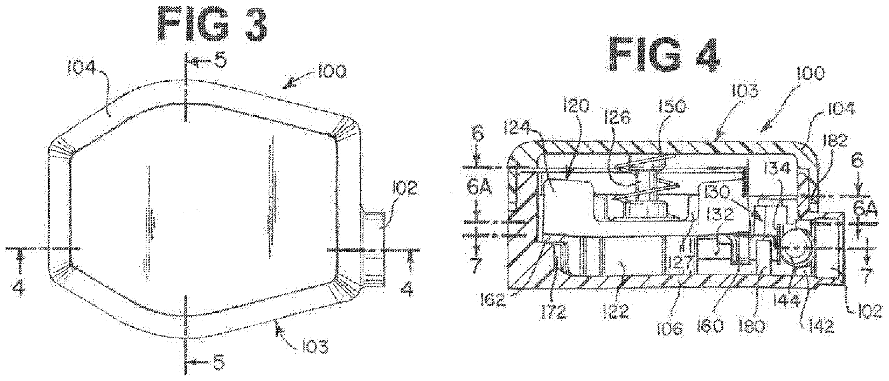

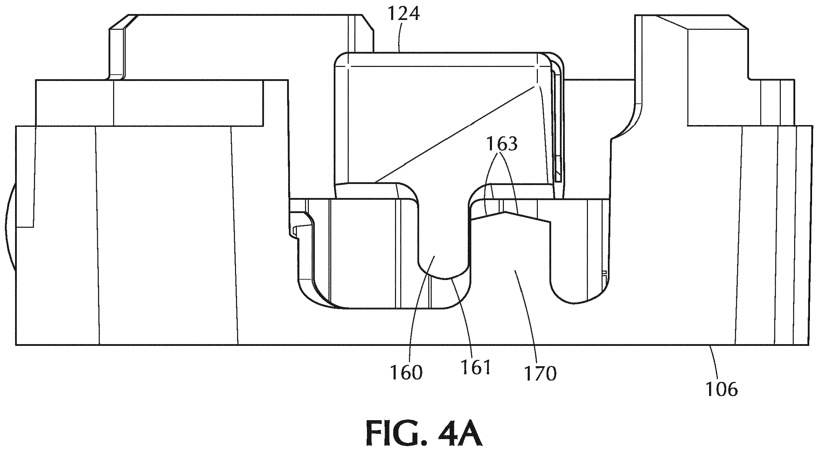

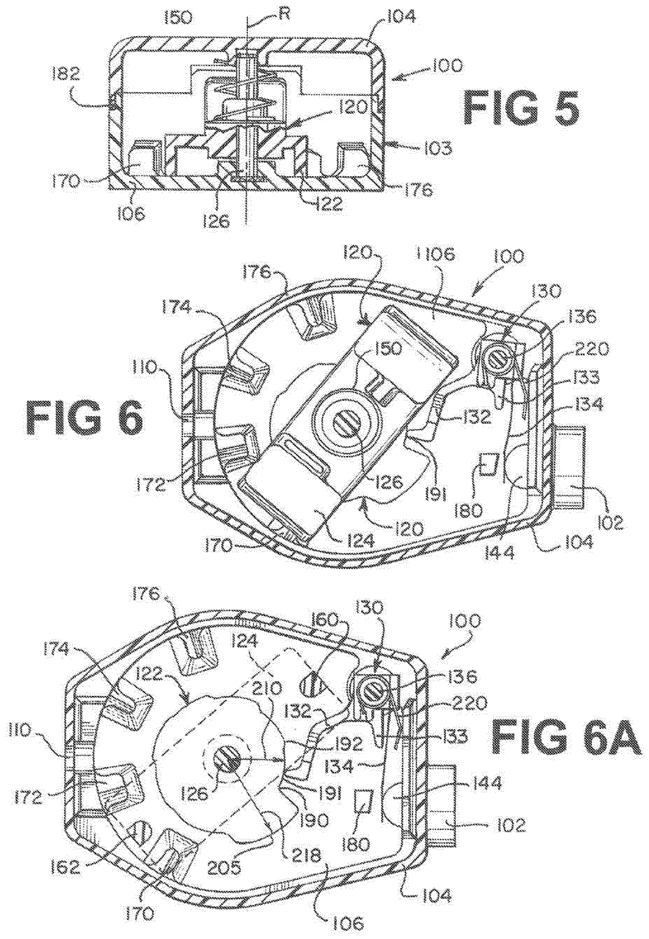

Adjustable valve unit 100 is shown assembled in Figures 3-5 and positioned at a second pressure setting, as described in more detail below. Rotor housing 124 carries downwardly projecting teeth 160 and 162 with cooperate with four lock stops 170, 172, 174, 176 projecting upwardly from lower casing 106 in this construction. Lock stop 172 is shown in partial cross-section in Figure 4 and lock stops 170 and 176 are visible in Figure 5. Preferably, the lower surfaces 161 of rotor teeth 160 and 162 are rounded and the upper surfaces of casing lock stops 170, 172, 174 and 176 each have a plurality of facets 163 to create a chisel-like, lead-in topography which encourages the rotor teeth to return to a constrained position, as illustrated in the side view in Figure 4A. However, the vertical surfaces of the rotor teeth 160, 162 and of lock stops 170-176 abut when engaged and do not "lead out", that is, relative translational movement is discouraged, once again illustrated in Figure 4A. Pure vertical lift must therefore be provided by an adjustment tool, as described in more detail below, to overcome the rotor teeth-to-lock stop abutment and change the performance setting.

A limiter 180, Figure 4, restricts travel of spring 134 away from seat 142 so that ball 144 does not become misaligned or dislodged relative to seat 142. A gasket 182 of epoxy is shown in Figures 4 & 5 as an optional, redundant seal between upper casing 104 and lower casing 106 in this construction.

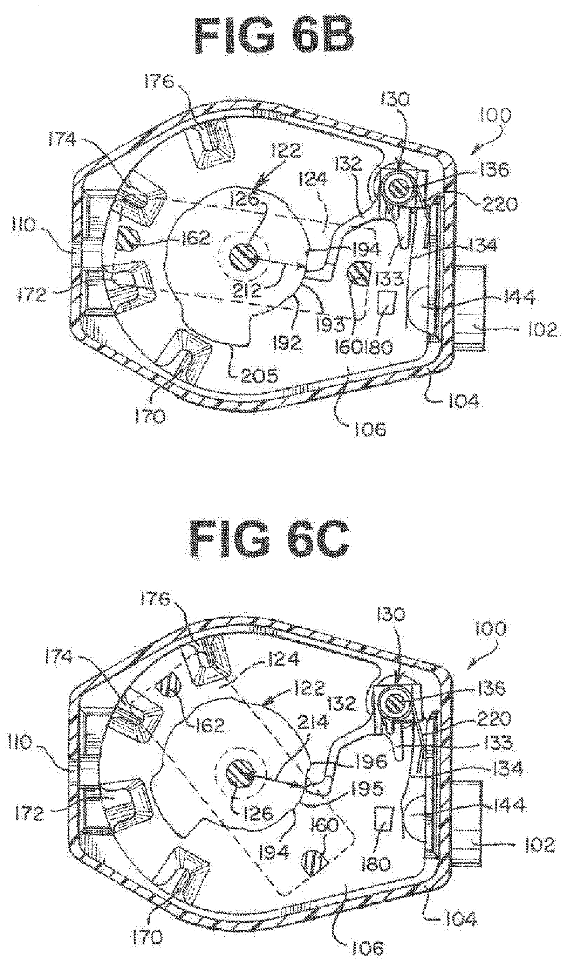

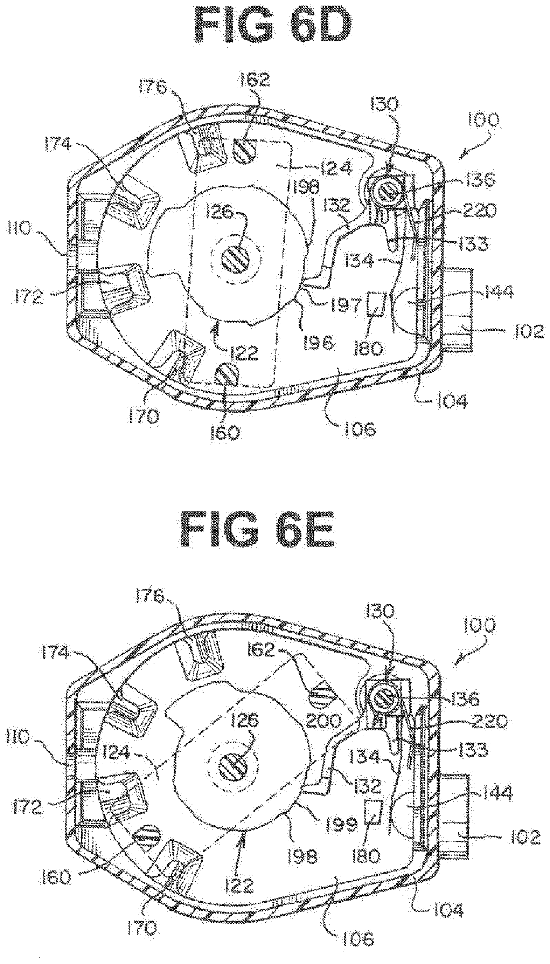

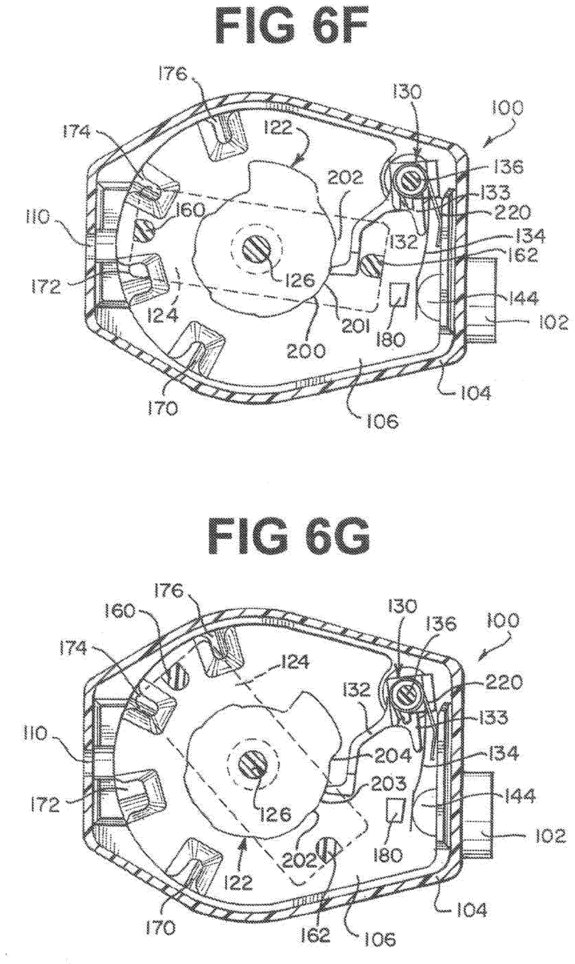

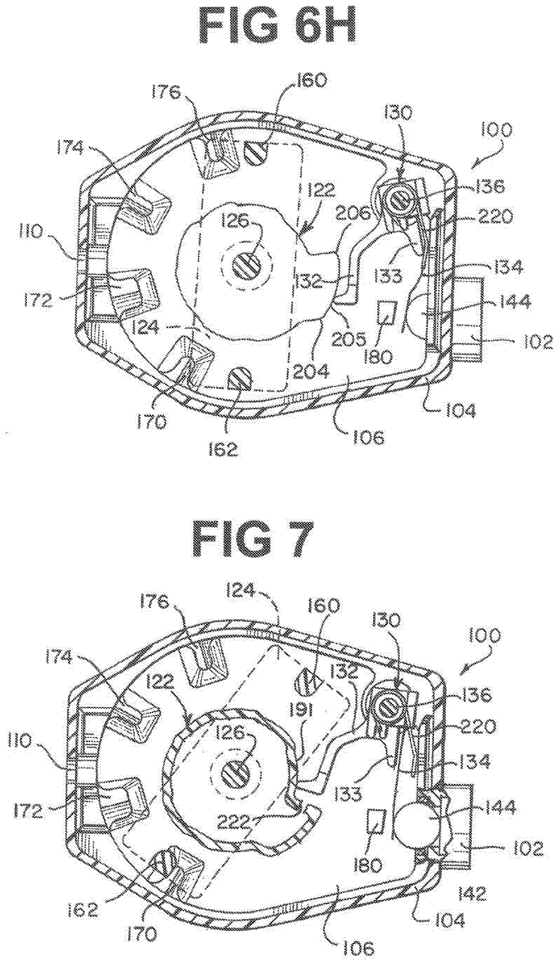

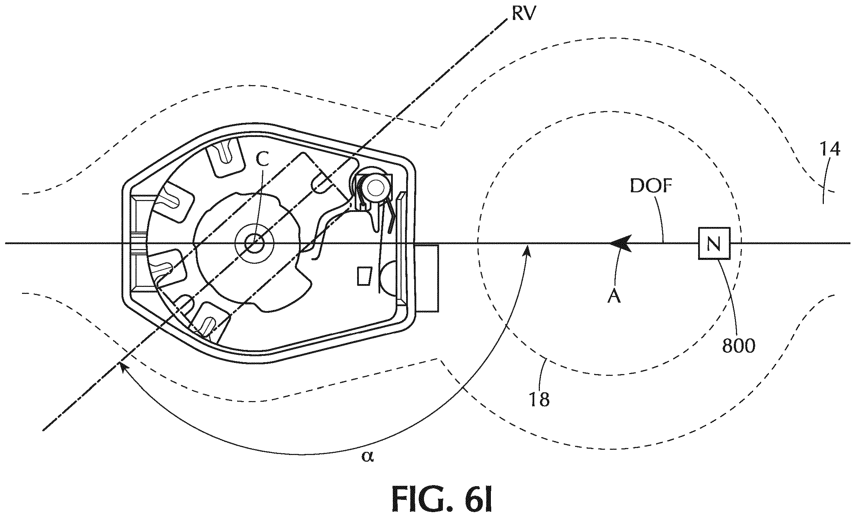

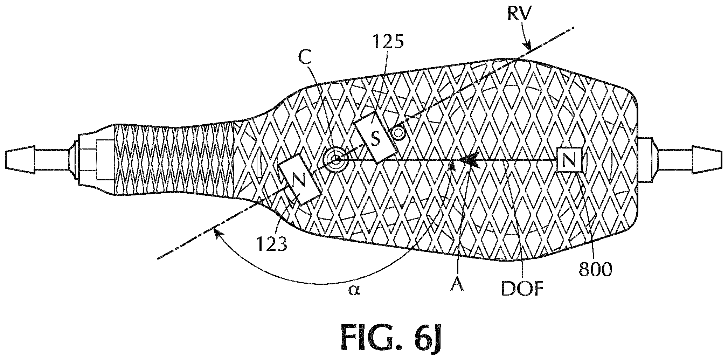

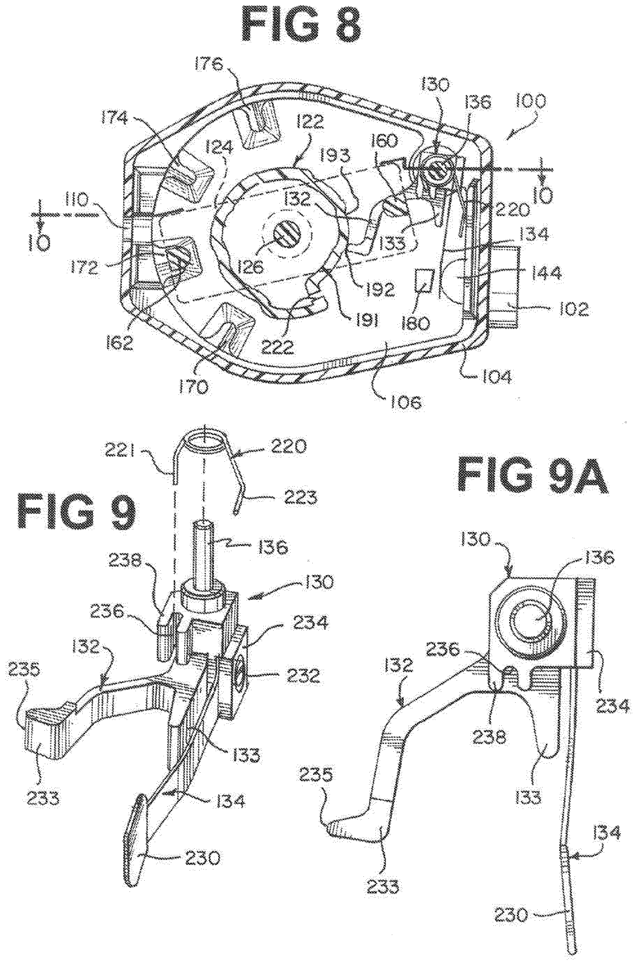

The operation of adjustable valve unit 100 is illustrated in Figures 6-8 with identical reference numerals identifying identical components and features. Not all such components and features are labelled in each drawing for the sake of visual clarity. Figures 6 & 6A show different levels of top partial cross-sectional views for adjustable valve unit 100 at a first pressure setting. Cam follower 132 slidably contacts only a first cam surface 191, which has an arc length bounded by points 190 and 192, because rotor housing tooth 162 is captured between casing lock stops 170 and 172 in the normal, constrained condition. First cam surface 191 has a first, preferably shortest radial distance 210 relative to the axis of rotation of rotor 120. By comparison, outermost cam surface 205 has a greatest radial distance 218. An optional torsion spring 220 is shown in greater detail in Figure 9.

When rotor 120 is translated upwardly by magnets using an adjustment tool rotor tooth 162 is lifted so that subsequent clockwise or counter-clockwise rotation of the adjustment tool rotates rotor tooth 162 up and over casing lock stop 172. After the adjustment tool is removed and when the second pressure setting has been selected as shown in Figure 6B, rotor 120 is biased downwardly by spring 150, Figures 2, 4 & 5.

Rotor tooth 160 is illustrated as not being in contact with any stop in Figures 4 & 6B, for example, because in the constrained condition rotor tooth 162 is now captured between a pair of adjacent lock stops 172 and 174, Figure 6B, which is sufficient to prevent rotation of rotor 120 relative to the cam follower 132 beyond points 192 and 194 on the cam structure of rotor 120. Points 192 and 194 represent a second arc length for second cam surface 193. Surface 193 is at a second radial distance 212 which is greater than distance 210 and is less than distance 218, Figures 6A & 6H. The arc length of second cam surface 193, Figure 6B, can be the same or different than the arc length of first cam surface 191 but, preferably, is substantially the same length.

The outward radial motion of cam follower 132 as it slidably travels from first cam surface 191, Figure 6A, to second cam surface 193, Figure 6B, increases the biasing force by valve spring 134 on ball 144 as increased torque is applied by cam follower 132 to the remainder of spring arm unit 130. Improved precision in pressure control is achieved by having a stiff cam follower 132 in contact with the selected cam surface and a flexible element, spring 134, in contact with the valve ball 144. The enhanced result is opening of the ball 144 from the valve seat 142 by requiring only the resilient spring element 134 to bend, which provides a constant spring force to the ball 144. The opening pressure, and overall valve performance, is not reliant on axial pivoting of the spring arm unit 130.

A third opening pressure setting is shown in Figure 6C with rotor tooth 162 positioned between casing stops 174 and 176 such that cam follower 132 experiences only third cam surface 195 between points 194 and 196 at a third radial distance 214. To achieve a fourth pressure setting, Figure 6D, both rotor teeth 160 and 162 are utilized relative to casing stops 170 and 176, respectively. Cam follower 132 is restricted thereby to fourth cam surface 197 between points 196 and 198.

Fifth through seventh pressure settings are illustrated in Figures 6E-6G as rotor tooth 160 is successively captured between casing adjacent lock stop pairs 170-172, 172-174, and 174-176, respectively. Cam follower 132 is restricted thereby to fifth cam surface 199 between points 198 and 200, Figure 6E, sixth cam surface 201 between points 200 and 202, Figure 6F, and seventh cam surface 203 between points 202 and 204, Figure 6G.

Preferred opening pressure settings currently range from approximately 30 mm to 210 mm water (294 Pa to 2,059 Pa) in seven increments of 30 mm (294 Pa), with a final, "virtual off' setting described in more detail below. Preferably, each valve unit is calibrated and tested at the time of manufacture at one or more flow rates. Actual opening pressure for each setting tends to vary according to flow rate, typically measured in milliliters per hour. Also, when tested with a 120 cm long distal catheter having an inner diameter of 1 mm, the average opening pressure typically will increase by 9 mm water or more at flow rates of 5 ml/h or more.

The final setting, Figure 6H, of approximately at least 400 mm water (3,920 Pa) minimizes flow as a "virtual off" setting, that is, as substantially closed. This final setting is achieved by exposing cam follower 132 to outermost cam surface 205, defined by points 204 and 206, having greatest radial distance 218. This greatest cam setting forces stiffener element 133 of spring arm unit 130 against valve spring 134 to shorten its active, effective length and thereby dramatically increase the biasing force applied against ball 144. The final opening pressure is increased by more than fifty percent over the prior setting. In other constructions, a stiffener element is forced against a valve spring during two or more final cam settings at desired pressure increments.

Spring arm unit 130 is shown in greater detail in Figures 9 and 9A with cam follower 132, stiffener element 133, and valve spring 134. Cam follower 132 terminates in a triangular head 233 with rounded or chamfered edges, one of which serves as a bearing surface 235. In a preferred construction, spring element 134 is formed from stainless steel having a thickness of 0.020 inches and terminates in an enlarged pad 230 for contacting the valve ball or other movable valve member. In one construction, spring element 134 is attached to the remainder of spring arm unit 130 by a post 232 and rivet 234 which are secured by ultrasonic welding. Torsion spring 220 has a first leg 221 which is retained in recess 236 of projection 238. Second spring leg 223 rests against an inner surface of the casing.

Use of torsion spring 220 is optional, and is possible because only spring element 134 contacts the movable valve member. As a result, additional spring force from torsion spring 220 can be utilized to force bearing surface 235 of cam follower 132 against a cam surface of the rotor. This biasing force provided by torsion spring 220 augments rotational position of the spring arm reflective of the intended cam displacement without otherwise impacting the force applied to the ball or other movable valve member. This provides for a more accurate and repeatable opening pressure and a more manufacturable and robust design as it reduces the need to maintain minimal friction such as when the valve spring element solely provides the force needed to maintain the cam follower on the cam surface.

The position of the components and features within adjustable valve unit 100 at the first pressure setting shown in Figure 6A is illustrated at a deeper partial cross-sectional view in Figure 7. Opening 222 into the lower cam portion of rotor 120 inhibits negative pressure from developing under rotor 120, that is, opening 222 ensures pressure equalization as cerebrospinal fluid passes through valve unit 100.

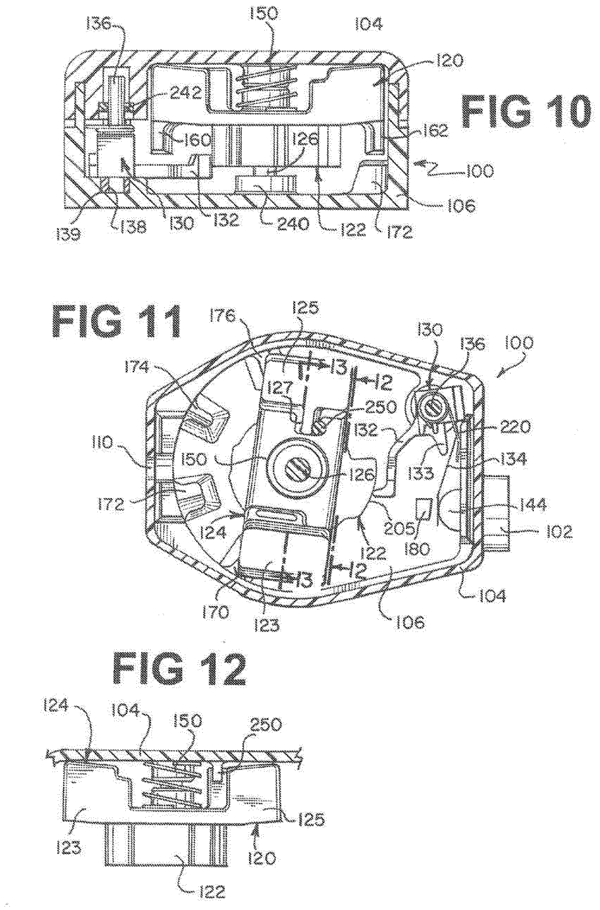

The transition from the first pressure setting to the second pressure setting is illustrated in Figures 8 & 10 as rotor 120 is translated upwardly by magnetic attraction with an adjustment tool so that rotor tooth 162 is able to clear casing lock stop 172. Cam follower 132 is shown in FIG. 8 at point 192 passing from first cam surface 191 to second cam surface 193. Lower cam section 122 has a sufficient height relative to cam follower bearing surface 235 to ensure that cam follower 132 remains in contact with a cam surface of cam portion 122 in both the constrained and unconstrained conditions. Rotor retention spring 150, Figure 10, has been compressed, its biasing force being overcome by magnetic attraction between rotor 120 and the adjustment tool while it is positioned over valve unit 100. Also illustrated in Figure 10 are upper and lower synthetic ruby bearings 242 and 139 for upper and lower axles 136 and 138, respectively, of spring arm unit 130. Synthetic ruby bearing 240 rotatably supports rotor axle 126.

The position of the components and features within valve unit 100 at the final, "virtual off' or substantially closed setting shown in Figure 6H is depicted at a shallower cross-sectional view in Figure 11 in an unconstrained condition. Further clockwise rotation of rotor 120 is prevented by rotation stop or limiter 250 which projects downwardly from upper casing 104 to contact finger 127. Rotation stop 250 contacts the opposite surface of finger 127 when rotor 120 is turned fully counter-clockwise in an unconstrained condition. The actual position of rotation stop 250 may be shifted to the right of the position shown in Figure 11 so that cam follower 132 is able to track nearly the entire portion of cam surface 205. Preferably, one side of stop 250 prevents rotor movement from the lowest setting directly to the highest setting, and also prevents the cam follower from touching the cam projection for the highest setting when the rotor is at its lowest setting. The other side of stop 250 prevents movement from the highest setting directly to the lowest setting. A side, partial cross-sectional view of rotation stop 250 blocking rotor housing 124, as well as spring 150 compressed between rotor 120 and upper casing 104, is shown in Figure 12 for this unconstrained condition.

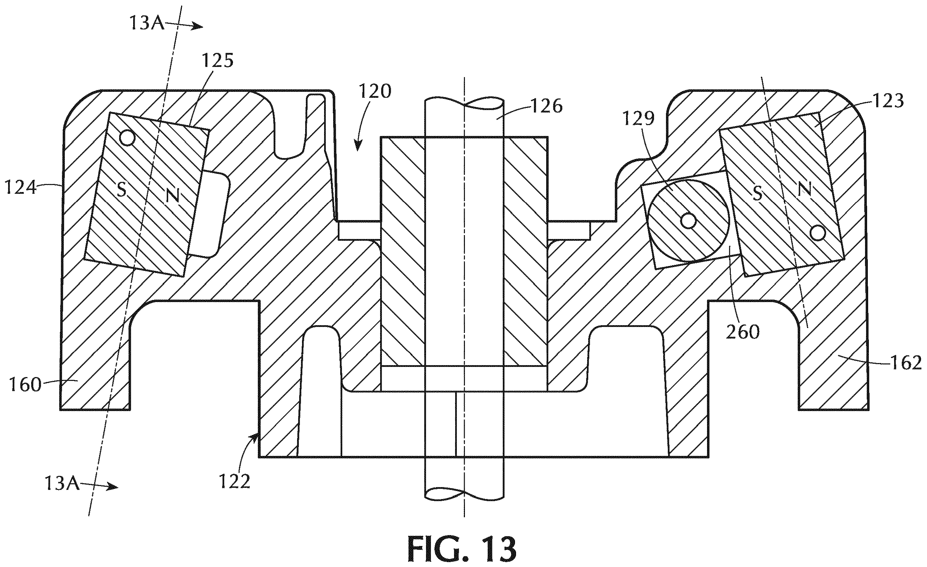

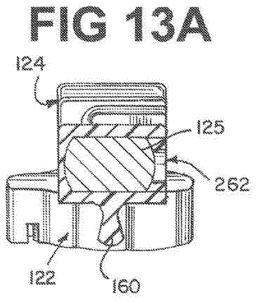

Further detailed views of selected features and components of rotor 120 in one construction are illustrated in Figures 13 & 13A. In particular, the housing portion 124 is shown as integral with cam portion 122, similar to monolithic rotor 120a of Figure 1A. Pocket cavity 260, Figure 13, contains magnet 123 and tantalum reference ball 129 which is readily visible during imaging of the valve unit 100 after implantation in a patient to confirm the actual pressure setting. Pocket cavity 262 holds magnet 125. A partial end view of housing portion 124 through magnet 125, pocket 262 and rotor tooth 160 is provided in Figure 13A.

It is therefore the particular design of the abutting rotor-tooth-to-lock-stop vertical surfaces requiring purely vertical lift by an adjustment tool to overcome the rotor-tooth-to-lock-stop abutment in order to change the pressure setting that provides the resistance to change of valve settings even during exposure to external foreign magnetic fields. The rotor tooth-to-lock stop abutment mechanically prevents rotational movement from a given performance setting as the vertical surfaces provide no lead in to facilitate travel up and over the lock stops made of a sufficiently rigid material (such as a moldable plastic, for example, polyethersulfone (PES), polysulfone (PSU), polyphenylsulfone (PPSU)) and sufficient wall thickness (greater than 0.2 mm) to prevent flexure. Axial movement is restricted due to the orientation of the rotating construct magnets inducing a combined attraction and repulsion when interacting with a strong north or south magnet. Furthermore, the interference between the axle and the bushing surface of the rotating construct mechanically limit tilt associated with attraction/repulsion to an external magnetic field.

However, it is only when the downward projecting rotor teeth 160, 162 of the rotor housing 124 are properly locked, engaged or seated in corresponding setting pockets 171, 171', 171", 171 ''' defined by at least one of the lock stops 170, 172, 174, 176 projecting upwardly from the lower casing 106 that the programmable implantable bodily fluid drainage valve is resistant to magnetic fields. It is mechanically possible for the downward projecting teeth 160, 162 when vertically lowered to undesirably rest on the lock stops 170, 172, 174, 176, as depicted in Figure 8 wherein rotor tooth 162 is resting on lock stop 172. When the rotor teeth 160, 162 are resting on the lock stops 170,172, 174, 176 (i.e., not properly seated in the respective setting pockets defined by the lock stops) the programmable implantable bodily fluid drainage valve is at risk of possible unwanted change to the valve setting when exposed to magnetic fields such as during an MRI procedure. Heretofore, conventional programming valves are not able to verify whether the rotor teeth 160, 162 are properly seated in the setting pockets 171, 171', 171", 171'''. Experimental testing has confirmed that the valve setting remains unchanged when subject to magnetic fields up to approximately 3T if the rotor teeth are properly locked, engaged or seated in the respective setting pockets. To be certain, prior to being exposed to a magnetic field (e.g., prior to undergoing an MRI procedure, the programmed valve setting must once again be verified by medical personnel using the indicator tool from the associated toolset or alternatively the medical personnel would have to use a tool to confirm proper engagement. The possibility of a change in valve setting if the rotor teeth are not properly seated in a setting pocket during exposure to magnetic fields, despite its relative small probability of occurrence, is still particularly problematic since the implantable programmable bodily fluid drainage valve cannot be guaranteed as being resistant to magnetic fields in such circumstances.

The present inventive improved implantable valve drainage system eliminates this uncertainty by verifying whether the downward projecting rotor teeth 160, 162 are properly seated in the respective seating pockets 171, 171', 171", 171''', that is, confirm whether the magnetic field resistance mechanism is properly engaged to carry out its intended functionality.

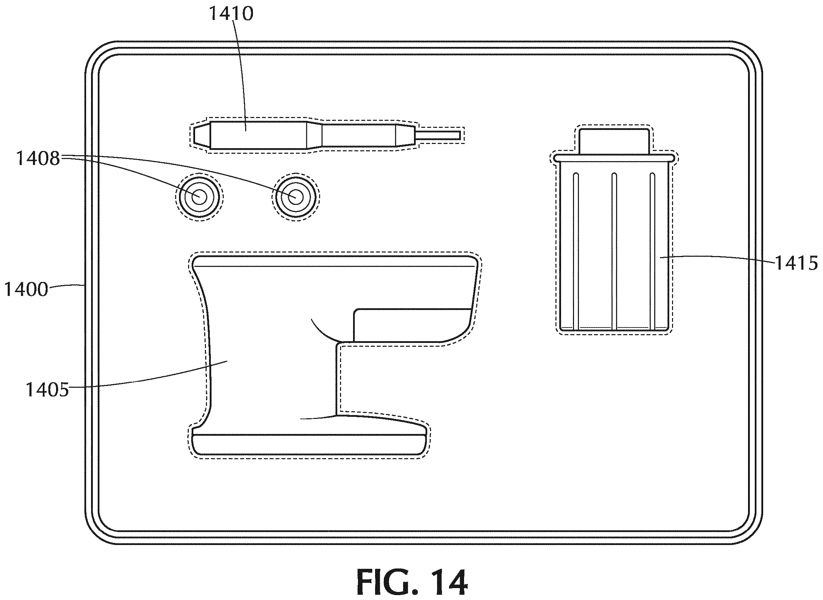

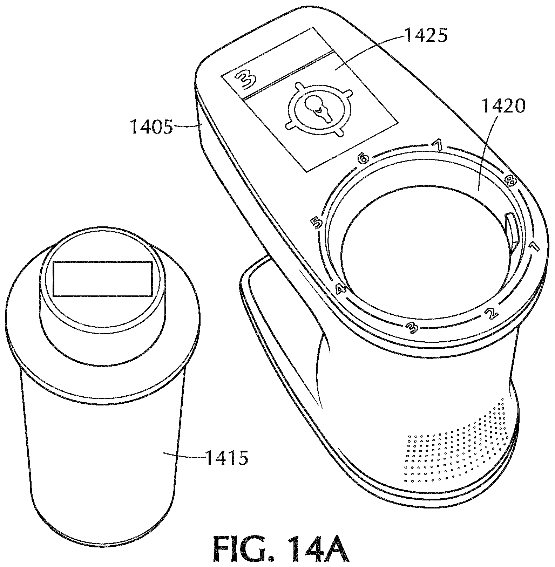

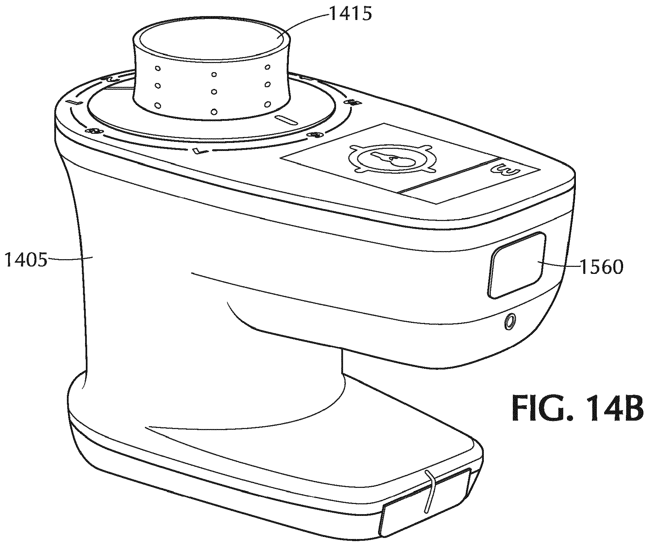

This is realized by configuring the programmable shunt valve 10 to include a fixed reference magnet 800, in addition to the primary magnetic elements 123, 125 disposed in the housing 124 of the rotor 120 of the adjustable valve unit, as illustrated in Figure 6I. Referring to Figure 6J, preferably, the fixed reference magnet 800 is located between the proximal connector 14 and the sampling/pumping chamber 18 within the direction of flow of the shunt valve. Preferably, fixed reference magnet 800 has a different magnetic strength from the primary magnetic elements 123, 125 and a different nominal distance between magnets (i.e., distance between reference magnet 800 and primary magnet 123 compared to distance between primary magnets 123 and 125) for proper identification. Nominal distance between primary magnetic elements 123, 125 is approximately 5.48mm as measured from bottom inner corner to bottom inner corner. Fixed reference magnet 800 nominal distance is 17.5mm from RC axle to leading edge of reference magnet 800. The fixed reference magnet 800 is aligned with an arrow indicia or marking "A" on the programmable shunt valve 10 itself denoting the direction of flow of fluid therethrough and a center point "C" midway between the magnetic elements 123, 125. A line passing through these three points (referred to as a direction of flow line) is the basis for determining the orientation of the programmable shunt valve 10 using an integrated locator/indicator tool 1405 from the exemplary tool set 1400 in a case, as illustrated in Figure 14. Also included in the tool set is an adjustment tool 1415, a screwdriver 1410 and spare batteries 1408. It is noted that the locator and indicator tools described herein and illustrated in the accompanying drawings have been integrated into a single device for simplicity of operation. It is, however, contemplated and within the intended scope of the present invention for none, some, or all of the tools in the toolset to be integrated.

A top perspective view of the integrated locator/indicator tool 1405 and adjustment tool 1415 of Figure 14, prior to the adjustment tool 1415 being inserted into a cavity 1420 of the integrated locator/indicator tool 1405, is shown in Figure 14A. While Figure 14B shows the adjustment tool 1415 following insertion into the cavity 1420.

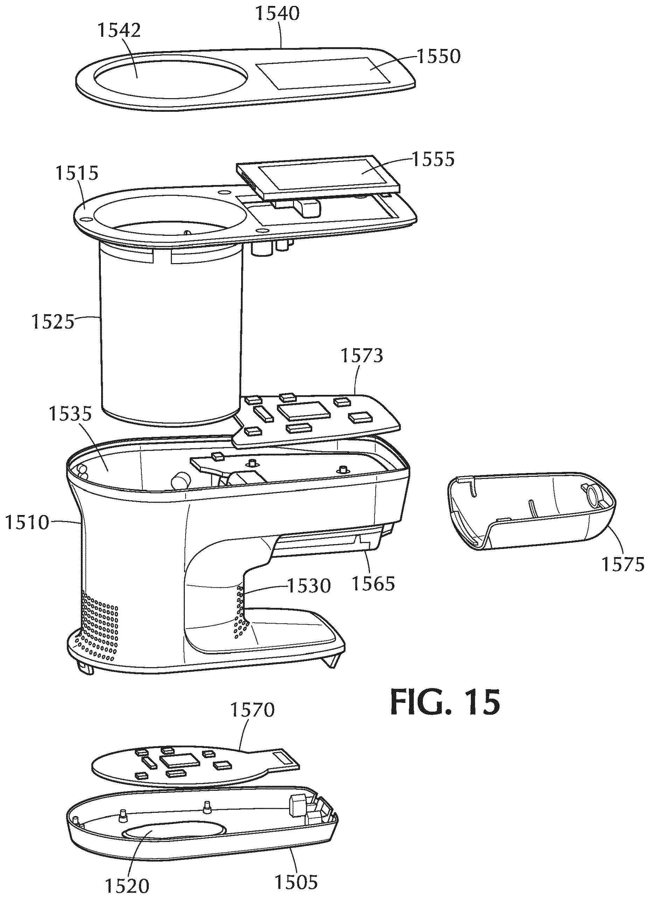

Figure 15 is an exploded perspective view of the integrated locator/indicator tool 1405 of Figure 14 which includes a housing 1500. In the illustrated example, housing 1500 comprises a bottom housing section 1505, a middle housing section 1510 and a top housing section 1515, each separate from one another. A cylindrical shaped section 1530 of the middle housing section 1510 defines a passageway or channel 1535 extending longitudinally therethrough. Top housing section 1515 has a chimney 1525 complementary in size and shape to be received within the passageway or channel 1535 of the cylindrical shaped section 1530 of the middle housing section 1510. Chimney 1525 is closed at one end and open at an opposite end. The open end of the chimney 1525 receiving therein the adjustment tool 1415, as described in detail below. An exterior surface of the bottom housing section 1505 has a recess 1520 defined therein that is complementary in shape and size to the outer contour of the programmable implantable bodily fluid drainage valve. In use, the integrated location/indication tool 1405 is positioned with the exterior surface of the bottom housing section 1505 against the skin of the patient and the implantable bodily fluid drainage valve seated within the recess 1520. A top covering or layer 1540 may be mounted to the top of the assembled housing. Such covering or layer 1540 has a complementary size and shape opening 1542 to that of the chimney 1525. Disposed about the perimeter of the opening 1542 are a series of markings representing the different valve settings in predetermined increments (e.g., 1, 2, 3, ,5, 6, 7, 8). A second opening 1550 in the top covering or layer 1540 permits viewing therethrough of a display 1555, such as a Liquid Crystal Display (LCD). The integrated locator/indicator tool 1405 is powered by one or more batteries and turned ON/OFF by a button 1560. The batteries are housed within a battery enclosure assembly 1565 that includes a tray with electronic contact terminals between which the batteries are inserted. Access to the battery enclosure assembly 1565 for insertion/removal of the batteries therefrom is via a removeable battery door assembly 1575. A two-dimensional array of 3-axis magneto-resistive sensors 1570 printed on a circuit board detects the magnetic field pattern produced by the magnetic elements 123, 125 disposed in the housing 124 of the rotor 120 and the fixed reference magnet 800. It is within the intended scope of the present invention to substitute other types of sensor arrays capable of detecting magnetic fields, such as Hall sensors, for the 3-axis magneto-resistive sensors 1570. Another printed circuit board 1573 includes a processor/controller and memory device.

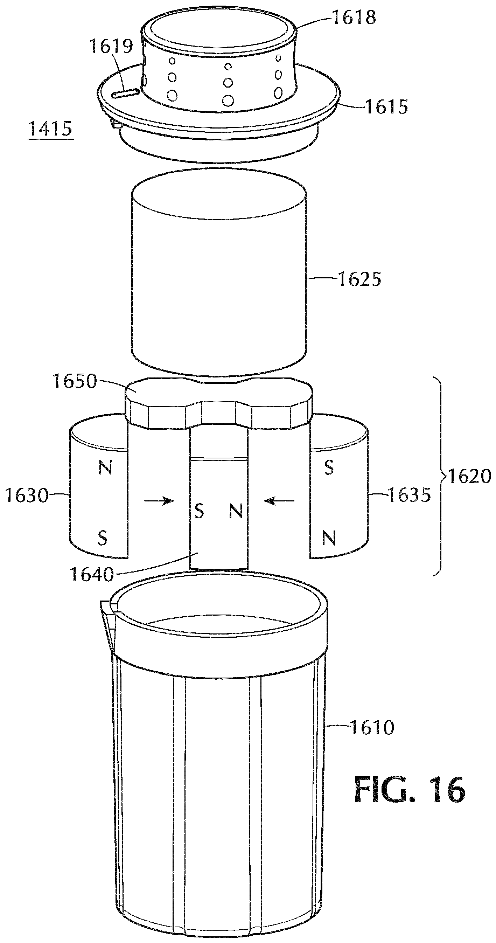

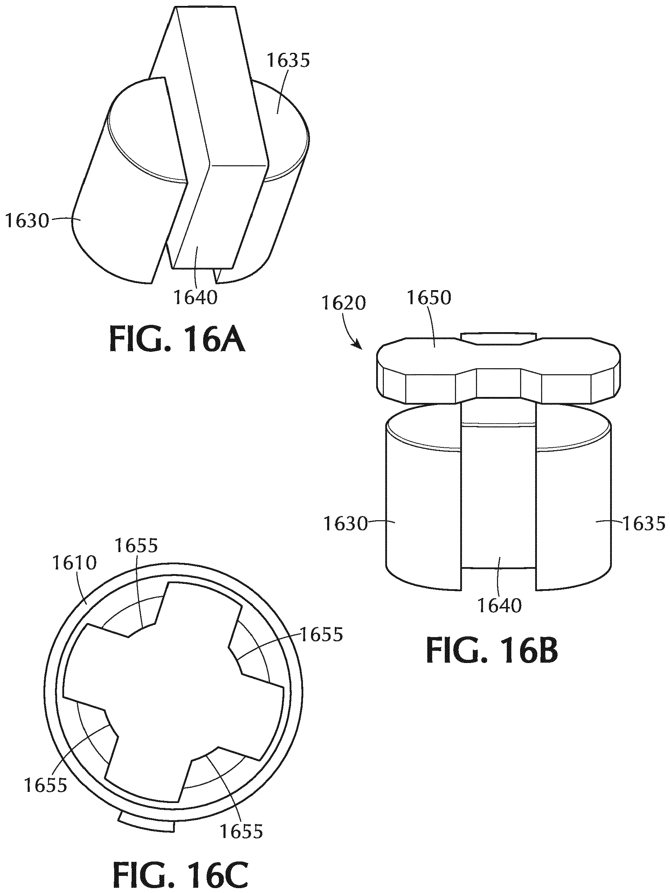

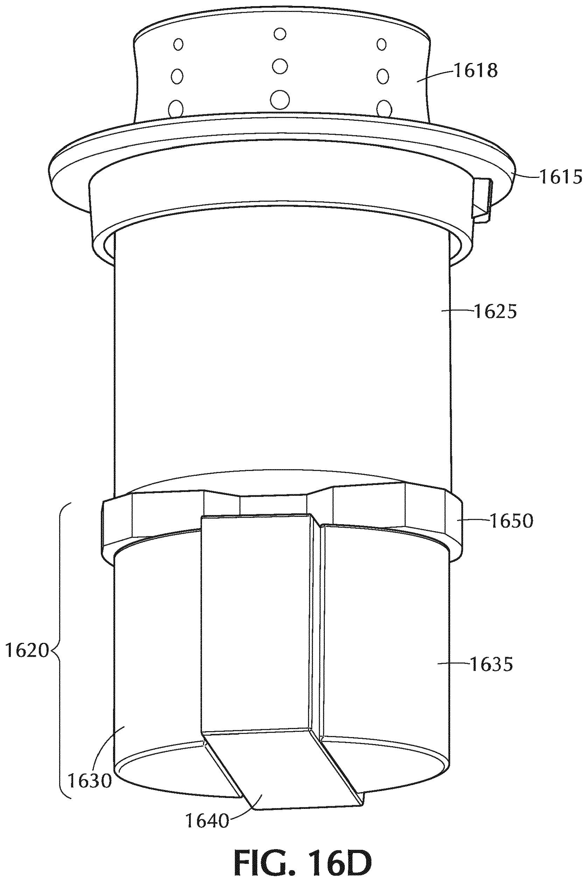

Figure 16 is an exploded perspective view of the adjustment tool 1415 of Figure 14. In the illustrated example, housing 1600 comprises an outer housing section 1610 and a top housing section 1615, each separate from one another. A magnet assembly 1620 is disposed in the outer housing section 1610. In particular, the magnet assembly 1620 in Figure 16A is a Halbach array comprising two half round magnets 1630, 1635 connected by a yoke 1650 and separated by a shield magnet 1640 that redirects the magnetic field allowing deeper penetration. The strength of the half round magnets 1630, 1635 selected for use in the adjustment tool 1415 depends on one or more factors, such as distance from the valve and the design of the sensor array. In the magnet assembly 1620, the two half round magnets 1630, 1635 are rotated until their flat side lays flush against the shield magnet 1640, as depicted in Figure 16A. The orientation of the magnets 1640, 1630, 1635 should preferably be as shown in Figure 16 with the magnet north side of the shield magnet 1640 in contact with the half round magnet 1630, 1635 with a magnetic north pointed toward the bottom of the outer housing section 1610. One of the two half round magnets 1630, 1635 faces the tantalum reference ball 129 (Figure 13). The shield magnet 1640 is partially repelled by the half round magnets 130, 1635 and thus is held down by a yoke 1650 mounted on top of the shield magnet 1640 that, when assembled, is also in contact with the two half round magnets 1630, 1635. It is these components of the magnet assembly 1620 that when assembled together are inserted into the outer housing section 1610 so that the two half round magnets 1630, 1635 are received in respective recesses 1655 defined in an interior surface of the outer housing section 1610 with the half round magnet facing the tantalum ball 129 facing towards the '1 to 8 stop'. As is visible in the top view in Figure 16C, the outer housing section 1610 includes a plurality of vertical ribs 1655 with which the half round magnets 1630, 1635 connect. A cylindrical shaped spacer 1625 is positioned above the yoke 1650 (Figure 16D). The top housing section 1615 with a marking indicator is secured to the outer housing section 1610 forming the assembled adjustment tool 1415.

The magnetic field pattern produced by the magnetic elements 123, 125 disposed in the housing 124 of the rotor 120 and the fixed reference magnet 800 is detected by the two-dimensional array of 3-axis magneto-resistive sensors 1570 of the integrated locator/indicator tool 1405. Once these three magnets are detected, the center point "C" midway between the two detected magnetic elements 123, 125 is located. The detected fixed reference magnet 800 is connected with the arrow indicia or marking "A" denoting the direction of flow on the implantable valve and the center point "C" midway between the two detected magnetic elements 123, 125 to define a direction flow line as a reference line for aligning the integrated locator/indicator tool 1405 with the direction of flow line on the valve. Once the user has centered and oriented the toolset over the valve mechanism the toolset will provide an indication of valve setting based on the angle of north/south poles and facilitate adjustment of the valve setting.

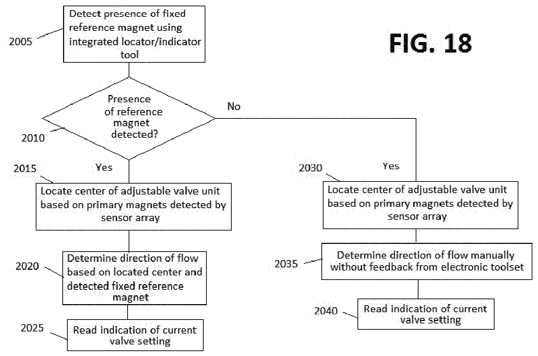

Figures 18A-18I are sequential steps in operation of the improved electronic toolset of Figure 14 in accordance with the present invention. In Figure 18A the integrated locator/indictor tool 1405 is powered on by pressing the power button 1560. Holding the power button 1560 for a predetermined period of time, e.g., approximately 3 seconds, calibrates, clears or zeros out the integrated locator/indicator tool 1405, as illustrated in Figure 18B. Then a bottom surface (sensor floor) of the integrated locator/indicator tool 1405 is positioned against the skin above the implantable valve system such that the implantable valve is received in the complementary size and shaped recess 1520 defined in the exterior surface of the bottom housing section 1505, as illustrated in Figure 18C. The integrated location/indication tool 1405 is moved in the appropriate direction (as indicated by the four arrows pointing in different directions) until the two circular visual images viewed on the LCD display 1555 are aligned with one another, indicating that the center of the adjustable valve unit 100 has been aligned with the center of the adjustable valve unit 100. Having centered the locator/indictor tool 1405 above the adjustable valve unit 100, then in Figure 18D, the integrated locator/indicator tool 1405 is rotated until the two visual icons (complementary in shape (key hole shaped) to the implantable valve) displayed within the two circular visual images are aligned with one another to orient the integrated location/indication tool 1405 in the proper direction of flow of the implantable valve. It is now that the integrated location/indication tool 1405 has been centered and oriented in a direction of flow of the implantable valve, that the current indication or valve setting is read and visually displayed on the LCD (Figure 18E). If the current valve setting is to be changed or programmed to a new valve setting, then in Figure 18F the adjustment tool 1415 is inserted into the cavity 1420 of the integrated location/indication tool 1405 and rotated until the reference marking 1619 on the adjustment tool 1415 is aligned with the marking on the top lens 1540 corresponding to the read current valve setting. In Figure 18G the adjustment tool 1415 is rotated clockwise/counterclockwise to the marking on the top lens corresponding to the new valve setting. Once set to the new valve setting, in Figure 18H the adjustment tool 1415 is removed from the integrated locator/indicator tool 1405 (while the integrated location/indication tool 1405 remains stationary in place) and this new valve setting is now automatically detected by the integrated location/indication tool 1405 and visibly displayed on the LCD 1555 (Figure 18I). It is noted that the positioning of the integrated location/indication tool 1405 remains unchanged in steps 18E-18I. The improved electronic toolset eliminates the requirement or need to have to once again locate the center of the valve and then confirm the new valve setting following adjustment by the adjustment tool 1415.

The present inventive electronic toolset, as illustrated in Figure 14, is suitable for use with a latest version or generation of the programmable implantable valve (Figure 1) having a fixed reference magnet 800 separate from and in addition to the pair of primary magnets 123, 125 in the adjustable valve unit 100. As discussed above, the fixed reference magnet is used to determine the angular orientation of the valve. Other, possibly older, generations or versions of the programmable implantable valve not employing a reference magnet separate from and in addition to the pair of primary magnets in the adjustable valve unit, may still be in use. Then the angular orientation of the valve cannot be ascertained using the electronic toolset. Of course, a generation or version of the programmable implantable valve not employing a reference magnet may still be programmed using its corresponding older version or generation of toolset (i.e., one that is not adapted to detect the fixed reference magnet and based on such determine electronically the angle of orientation). However, this would require medical personnel to have available on site different versions or generations of electronic toolsets corresponding to whether the fixed reference magnet is present in the programmable implantable valve or not. Selecting the appropriate generation or version of toolset would first require the user to identify which version or generation of the implantable programmable valve was implanted. This may be accomplished via X-ray imaging (e.g., identifying the presence or absence of a reference magnet), however, such exposure has deleterious health effects and thus is to be avoided, whenever possible. Another drawback is that medical facilities would require an allocation of space for storing of the different versions or generations of toolsets. However, by far one of the most relevant risks is the possible selection and use by medical personnel of an incompatible generation or version of toolset with the implanted valve. These risks and drawbacks are reduced or overcome by using the present inventive universal electronic toolset that may be interchangeably used with both programmable implantable valves employing a fixed reference magnet as well as with earlier generations or versions of programmable implantable valves that do not include a fixed reference magnet.

Specifically, the present inventive locator/indicator tool 1405 includes circuitry for determining whether the fixed reference magnet 800 associated with the adjustable valve unit 100 is detected by the 2-dimensional array of 3-axis magneto-resistive sensors 1570. If the fixed reference magnet 800 is not detected (i.e., not present) the system classifies the magnets based on the historical valve mechanism and facilitates guidance for locating the valve but requires the user to determine orientation without electronic assistance or feedback, such as through palpation. Once the user has manually oriented the toolset over the valve mechanism the toolset will provide an indication of valve setting based on the angle of north/south poles and facilitate adjustment of the valve setting.

Figure 18 is an exemplary flow chart of the specific steps taken to ascertain whether the implanted valve 10 is a generation or version that includes a fixed reference magnet 800 or not and associated operational steps taken in either case. Specifically, in step 2005 the presence of the fixed reference magnet 800 in the implanted valve 10 is ascertained using the two-dimensional array of 3-axis magneto-resistive sensors 1570 in the integrated locator/indicator tool 1405. If the fixed reference magnet 800 in the implanted valve 10 is detected in step 2010, then the center of adjustable valve unit 100 is located in step 2015. Specifically, locating the center of the adjustable valve unit 100 is achieved by first detecting the primary magnets 123, 125 associated with the adjustable valve unit 100 using the two-dimensional array of 3-axis magneto-resistive sensors 1570 in the integrated locator/indicator tool 1405. Once the primary magnets 123, 125 have been detected, the center of the adjustable valve unit 100 is located midway therebetween representing the center of the adjustable valve unit 100. Next, in step 2020 the direction of flow of the valve is determined based on the located center of the adjustable valve unit 100 (in step 2015) and the detected fixed reference magnet 800 (in step 2005). The integrated location/indication tool 1405 is moved until aligned with the identified center of the adjustable valve unit 100 and then the tool is rotated to the proper orientation aligned with the identified direction of flow of the valve. Preferably, this is accomplished visually on a display screen 1555 on which are displayed two separate icons (one fixed valve location icon illustrative of the implanted valve and one movable locator/indicator tool icon representing the integrated location/indication tool 1405). Note that a single icon can be used to denote more than one parameter (e.g., centering and direction of flow) hereinafter referred to as a "dual parameter icon." For example, the dual parameter icon may be an outer circle with a key-hole shape within the outer circle complementary to the outline of the programmable shunt valve device 10 in Figure 1. In this exemplary dual parameter icon, the outer circle is representative of the centering parameter, whereas the key-hole shape within the outer circle denoting the direction of flow parameter. Integrated locator/indicator tool 1405 is moved and rotated appropriately until the two icons visible on the display screen 1555 are aligned relative to both the center (outer circles aligned) and direction of flow of the valve (key-hole shape). Now that the integrated location/indication tool 1405 is properly centered and the angle of orientation aligned with the direction of flow of the valve, the operation advances to step 2025 wherein the indication of the current valve setting is read by the integrated locator/indicator tool 1405 based on the angle of north/south poles of the primary magnets 123, 125 and facilitate adjustment of the valve setting, if desired.

If the new electronic toolset is being used with a previous generation or version of an implanted valve not employing a fixed reference magnet, then the presence of such fixed reference magnet will not be detected by the two-dimensional array of 3-axis magneto-resistive sensors 1570 in step 2010. The processing advances to step 2030 wherein the center of adjustable valve unit 100 is located in a manner similar to the processing discussed above with respect to step 2015. Specifically, locating the center of the adjustable valve unit 100 is achieved by first detecting the primary magnets 123, 125 associated with the adjustable valve unit 100 using the two-dimensional array of 3-axis magneto-resistive sensors 1570 in the integrated locator/indicator tool 1405. Once the primary magnets 123, 125 have been detected, the center of the adjustable valve unit 100 is located midway therebetween. The integrated location/indication tool 1405 is moved until aligned with the identified center of the adjustable valve unit 100. Once again this is preferably accomplished visually on the display screen 1555 on which are displayed two separate icons (one fixed valve location icon illustrative of the implanted valve and one movable locator/indicator tool icon representing the integrated location/indication tool 1405). When the presence of the fixed reference magnet is not detected, the icon displayed (e.g., the outer circle only) represents a single parameter (e.g., centering). There is no key-hole shape within the outer circle denoting the direction of flow parameter since such parameter is not detectable electronically with the assistance and feedback of the integrated locator/indicator tool 1405 in the absence of the fixed reference magnet 800. Integrated locator/indicator tool 1405 is moved appropriately until the two icons visible on the display screen 1555 are aligned relative to the center (outer circles aligned). Since no fixed reference magnet has been detected and thus no electronic assistance or feedback information regarding the direction of flow line of the implanted valve is provided by the integrated locator/indicator tool 1405, instead the operator is informed (via one or more senses such as visually, audibly and/or tactile) that the center and direction of flow, i.e., angle of orientation, of the adjustable valve unit must be determined manually without any electronic assistance or feedback information from any tool in the electronic toolset. Preferably, the user is guided step-by-step how to manually orient the toolset over the implanted valve, that is manually determine the center and direction of flow of the implanted valve (step 2035), without any electronic assistance or feedback information provided by the electronic toolset. By way of illustrative example based on the adjustable valve unit 100 configuration illustrated, the user may be instructed to palpate the area of interest until the adjustable valve unit 100 is located and then the center is marked (i.e., the hard portion of the valve distal to the reservoir). The position of the inlet and outlet connector barbs on the respective catheters may also be determined through palpation and marked accordingly. A straight line passing through the three markings then represents the direction of flow line. Once the direction of flow line has been ascertained manually (without any electronic assistance or feedback information from any tool in the electronic toolset) the integrated locator/indicator tool 1405 is manually oriented to align with the direction of flow line. Now that the center of the adjustable valve unit 100 has been located and the integrated location/indication tool 1405 has been manually aligned with the manually detected orientation of the direction of flow of the implanted valve, the operation advances to step 2040 wherein the indication of the current valve setting is read by the integrated locator/indicator tool 1405 based on the angle of north/south poles of the primary magnets 123, 125 and facilitate adjustment of the valve setting, if desired. As previously mentioned, with other valve configurations the steps for locating the center and direction of flow of the valve may vary from those described above using other fixed reference points on the valve.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A method for using a universal electronic toolset for indicating and adjusting of an implantable programmable bodily fluid drainage valve whether the implantable programmable bodily fluid drainage valve includes a fixed reference magnet used to determine an angle of orientation of the implantable programmable bodily fluid drainage valve or not, wherein the implantable programmable bodily fluid drainage valve includes an adjustable valve unit having a pair of primary magnetic elements, the method comprising the steps of:
detecting using a magnetic field detection sensor array in an indicator tool of the electronic toolset whether the fixed reference magnet is present in the implantable programmable bodily fluid drainage valve;
wherein if the presence of the fixed reference magnet in the implantable bodily fluid drainage valve is detected, further comprising the steps of:
locating a center of the adjustable valve unit using the indicator tool of the electronic toolset;
ascertaining a direction of flow of the adjustable valve unit based exclusively on electronic feedback provided by the indicator tool of the electronic toolset, without requiring manual physical palpation;
aligning the indicator tool of the electronic toolset with the located center and the direction of flow of the adjustable valve unit;
reading a current valve setting using the indicator tool of the electronic toolset;
wherein if the presence of the fixed reference magnet in the implantable programmable bodily fluid drainage valve is not detected, further comprising the steps of:
locating the center of the adjustable valve unit using the indicator tool of the electronic toolset;
establishing the direction of flow of the adjustable valve unit exclusively by manual physical palpation, without electronic feedback from the indicator tool of the electronic toolset;
aligning the indicator tool of the electronic toolset with the located center and the direction of flow of the adjustable valve unit;
reading the current valve setting using the indicator tool of the electronic toolset.

2. The method according to claim 1, wherein each of the locating steps comprises the steps of:
detecting using the magnetic field detection sensor array a magnetic field pattern produced by each of the pair of primary magnetic elements; and
finding a midway point between the detected pair of primary magnetic elements representing the center of the adjustable valve unit.

3. The method according to claim 1, wherein the direction of flow of the adjustable valve unit in the ascertaining step represents a reference line intersecting with: (i) an indicia denoting direction of flow of fluid through the implantable bodily fluid drainage valve; (ii) the found midway point between the detected pair of primary magnetic elements; and (iii) the detected fixed reference magnet.

4. The method according to claim 1, wherein the establishing step comprises the step of, exclusively via manually physically palpating an area of interest, locating a center and a direction of flow of the adjustable valve unit.

5. An implantable programmable bodily fluid drainage valve system comprising:
an implantable programmable bodily fluid drainage valve having an adjustable valve unit including a pair of primary magnetic elements for programming the implantable programmable bodily fluid drainage valve to a desired valve setting;
a universal electronic toolset for indicating and adjusting the implantable programmable bodily fluid drainage valve; wherein the universal electronic toolset comprises:
an indicator tool having a magnetic field detection sensor array for: (i) detecting the pair of primary magnetic elements; and (ii) determining the presence or absence of a fixed reference magnet in the implantable programmable bodily fluid drainage valve;
circuitry for determining a center of the adjustable valve unit based on the detected pair of primary magnetic elements; the circuitry determining exclusively by electronic feedback from the toolset an angular orientation of the implantable programmable bodily fluid drainage valve, when the fixed reference magnet is present; whereas the circuitry generating on a display of the implantable programmable bodily fluid drainage valve steps for determining exclusively by manual physical palpation the angular orientation of the implantable programmable bodily fluid drainage valve, when the fixed reference magnet is absent.

6. The system according to claim 5, wherein the angular orientation is a direction of flow of fluid through the implantable programmable bodily fluid drainage valve.

7. The system according to claim 5, wherein the circuitry determines the center of the adjustable valve unit by:
detecting using the magnetic field detection sensor array a magnetic field pattern produced by each of the pair of primary magnetic elements; and
finding a midway point between the detected pair of primary magnetic elements representing the center of the adjustable valve unit.

8. The system according to claim 7, wherein when the fixed reference magnet is detected as being present, the circuitry determines exclusively by the electronic feedback from the toolset the angular orientation of the implantable programmable bodily fluid drainage valve representing a reference line intersecting with: (i) an indicia denoting direction of flow of fluid through the implantable bodily fluid drainage valve; (ii) the found midway point between the detected pair of primary magnetic elements; and (iii) the detected fixed reference magnet.

## Patentansprüche

1. Verfahren zur Verwendung eines universellen elektronischen Werkzeugsatzes zum Anzeigen und Einstellen eines implantierbaren programmierbaren Körperflüssigkeitsdrainageventils, unabhängig davon, ob das implantierbare programmierbare Körperflüssigkeitsdrainageventil einen festen Referenzmagneten aufweist, der zum Bestimmen eines Orientierungswinkels des implantierbaren programmierbaren Körperflüssigkeitsdrainageventils verwendet wird oder nicht, wobei das implantierbare programmierbare Körperflüssigkeitsdrainageventil eine einstellbare Ventileinheit mit einem Paar primärer magnetischer Elemente aufweist, wobei das Verfahren die folgenden Schritte umfasst:
Detektieren unter Verwendung eines Magnetfelddetektionssensorarrays in einem Anzeigewerkzeug des elektronischen Werkzeugsatzes, ob der feste Referenzmagnet in dem implantierbaren, programmierbaren Körperflüssigkeitsdrainageventil vorhanden ist;
wobei, wenn das Vorhandensein des festen Referenzmagneten in dem implantierbaren Körperflüssigkeitsdrainageventil detektiert wird, ferner die folgenden Schritte umfasst:
Lokalisieren eines Zentrums der einstellbaren Ventileinheit unter Verwendung des Anzeigewerkzeugs des elektronischen Werkzeugsatzes;
Feststellen einer Durchflussrichtung der einstellbaren Ventileinheit, basierend ausschließlich auf einer elektronischen Rückmeldung, die von dem Anzeigegerät des elektronischen Werkzeugsatzes bereitgestellt wird, ohne dass ein manuelles physisches Abtasten erforderlich ist;
Ausrichten des Anzeigewerkzeugs des elektronischen Werkzeugsatzes auf das lokalisierte Zentrum und die Durchflussrichtung der einstellbaren Ventileinheit;
Ablesen einer aktuellen Ventileinstellung unter Verwendung des Anzeigewerkzeugs des elektronischen Werkzeugsatzes;
wobei, wenn das Vorhandensein des festen Referenzmagneten in dem implantierbaren, programmierbaren Körperflüssigkeitsdrainageventil nicht erkannt wird, ferner die folgenden Schritte umfasst:
Lokalisieren des Zentrums der einstellbaren Ventileinheit unter Verwendung des Anzeigewerkzeugs des elektronischen Werkzeugsatzes;
Festlegen der Durchflussrichtung der einstellbaren Ventileinheit ausschließlich durch manuelles Abtasten,
ohne elektronische Rückmeldung durch das Anzeigegerät des elektronischen Werkzeugsatzes;
Ausrichten des Anzeigewerkzeugs des elektronischen Werkzeugsatzes auf das lokalisierte Zentrum und die Durchflussrichtung der einstellbaren Ventileinheit;
Ablesen der aktuellen Einstellung des Ventils mit dem Anzeigewerkzeug des elektronischen Werkzeugsatzes.

2. Verfahren nach Anspruch 1, wobei jeder der Lokalisierungsschritte die folgenden Schritte umfasst:
Detektieren eines Magnetfeldmusters, das von jedem des Paares primärer magnetischer Elemente erzeugt wird, unter Verwendung des Magnetfelddetektionssensorarrays; und
Auffinden eines Mittelpunkts zwischen dem detektierten Paar von primären magnetischen Elementen, die das Zentrum der einstellbaren Ventileinheit repräsentieren.

3. Verfahren nach Anspruch 1, wobei die Durchflussrichtung der verstellbaren Ventileinheit im Feststellungsschritt eine Referenzlinie repräsentiert, die sich schneidet mit: (i) einem Anzeichen, das die Durchflussrichtung der Flüssigkeit durch das implantierbare Körperflüssigkeitsdrainageventil angibt; (ii) dem gefundenen Mittelpunkt zwischen dem detektierten Paar von primären magnetischen Elementen; und (iii) dem detektierten festen Referenzmagneten.

4. Verfahren nach Anspruch 1, wobei der Festlegungsschritt den Schritt umfasst, ausschließlich durch manuelles physisches Abtasten eines interessierenden Bereichs ein Zentrum und eine Durchflussrichtung der einstellbaren Ventileinheit zu lokalisieren.

5. Implantierbares, programmierbares Körperflüssigkeitsdrainageventil-System, umfassend:
ein implantierbares programmierbares Körperflüssigkeitsdrainageventil mit einer einstellbaren Ventileinheit, die ein Paar primärer magnetischer Elemente zum Programmieren des implantierbaren programmierbaren Körperflüssigkeitsdrainageventils auf eine gewünschte Ventileinstellung aufweist;
ein universelles elektronisches Werkzeugset zum Anzeigen und Einstellen des implantierbaren, programmierbaren Körperflüssigkeitsdrainageventils; wobei das universelle elektronische Werkzeugset umfasst:
ein Anzeigewerkzeug mit einem Magnetfelddetektionssensorarray zum: (i) Detektieren des Paares von primären magnetischen Elementen; und (ii) Bestimmen des Vorhandenseins oder Nichtvorhandenseins eines festen Referenzmagneten in dem implantierbaren, programmierbaren Körperflüssigkeitsdrainageventil;
eine Schaltungsanordnung zum Bestimmen eines Zentrums der einstellbaren Ventileinheit basierend auf dem detektierten Paar primärer magnetischer Elemente; wobei die Schaltungsanordnung ausschließlich durch elektronische Rückmeldung von dem Werkzeugsatz eine Winkelausrichtung des implantierbaren programmierbaren Körperflüssigkeitsdrainageventils bestimmt, wenn der feste Referenzmagnet vorhanden ist; obgleich die Schaltungsanordnung auf einer Anzeige des implantierbaren programmierbaren Körperflüssigkeitsdrainageventils Schritte zum Bestimmen der Winkelausrichtung des implantierbaren programmierbaren Körperflüssigkeitsdrainageventils ausschließlich durch manuelles physisches Abtasten erzeugt, wenn der feste Referenzmagnet nicht vorhanden ist.

6. System nach Anspruch 5, wobei die Winkelausrichtung eine Durchflussrichtung der Flüssigkeit durch das implantierbare programmierbare Körperflüssigkeitsdrainageventil ist.

7. System nach Anspruch 5, wobei die Schaltungsanordnung das Zentrum der einstellbaren Ventileinheit bestimmt, durch:
Detektieren eines Magnetfeldmusters, das von jedem des Paares primärer magnetischer Elemente erzeugt wird, unter Verwendung des Magnetfelddetektionssensorarrays; und
Auffinden eines Mittelpunkts zwischen dem detektierten Paar von primären magnetischen Elementen, die das Zentrum der einstellbaren Ventileinheit repräsentieren.

8. System nach Anspruch 7, wobei die Schaltungsanordnung, wenn das Vorhandensein des festen Referenzmagneten detektiert wird, ausschließlich durch die elektronische Rückmeldung vom Werkzeugsatz die Winkelausrichtung des implantierbaren, programmierbaren Körperflüssigkeitsdrainageventils bestimmt, die eine Referenzlinie repräsentiert, die sich schneidet mit: (i) einem Anzeichen, das die Durchflussrichtung der Flüssigkeit durch das implantierbare Körperflüssigkeitsdrainageventil angibt; (ii) dem gefundenen Mittelpunkt zwischen dem detektierten Paar von primären magnetischen Elementen; und (iii) dem detektierten festen Referenzmagneten.I

## Revendications

1. Procédé d'utilisation d'une boîte à outils électronique universelle pour indiquer et régler une valve de drainage de liquide corporel programmable implantable, que la valve de drainage de liquide corporel programmable implantable contienne un aimant de référence fixe servant à déterminer un angle d'orientation de la valve de drainage de liquide corporel programmable implantable ou non, dans lequel la valve de drainage de liquide corporel programmable implantable contient une unité de valve réglable possédant une paire d'éléments magnétiques primaires, le procédé comprenant les étapes consistant à :
détecter, au moyen d'un réseau de capteurs de détection de champ magnétique d'un outil indicateur de la boîte à outils électronique, si l'aimant de référence fixe est présent dans la valve de drainage de liquide corporel programmable implantable ;
le procédé, si la présence de l'aimant de référence fixe dans la valve de drainage de liquide corporel implantable est détectée, comprenant également les étapes consistant à :
localiser un centre de l'unité de valve réglable au moyen de l'outil indicateur de la boîte à outils électronique ;
déterminer une direction d'écoulement de l'unité de valve réglable exclusivement d'après la rétroaction électronique fournie par l'outil indicateur de la boîte à outils électronique, sans nécessiter de palpation physique manuelle ;
aligner l'outil indicateur de la boîte à outils électronique sur le centre localisé et la direction d'écoulement de l'unité de valve réglable ;
lire une valeur de paramètre de valve courante au moyen de l'outil indicateur de la boîte à outils électronique ;
le procédé, si la présence de l'aimant de référence fixe dans la valve de drainage de liquide corporel implantable n'est pas détectée, comprenant également les étapes consistant à :
localiser le centre de l'unité de valve réglable au moyen de l'outil indicateur de la boîte à outils électronique ;
établir la direction d'écoulement de l'unité de valve réglable exclusivement par palpation physique manuelle,
sans rétroaction électronique provenant de l'outil indicateur de la boîte à outils électronique ;
aligner l'outil indicateur de la boîte à outils électronique sur le centre localisé et la direction d'écoulement de l'unité de valve réglable ;
lire la valeur de paramètre de valve courante au moyen de l'outil indicateur de la boîte à outils électronique.

2. Procédé selon la revendication 1, dans lequel chacune des étapes de localisation comprend les étapes consistant à :
détecter, au moyen du réseau de capteurs de détection de champ magnétique, un motif de champ magnétique produit par chaque élément de la paire d'éléments magnétiques primaires ; et
trouver un point médian entre les éléments de la paire d'éléments magnétiques primaires détectée, représentant le centre de l'unité de valve réglable.

3. Procédé selon la revendication 1, dans lequel la direction d'écoulement de l'unité de valve réglable lors de l'étape de détermination représente une ligne de référence passant par : (i) un signe indiquant la direction d'écoulement de liquide à travers la valve de drainage de liquide corporel implantable ; (ii) le point médian trouvé entre les éléments de la paire d'éléments magnétiques primaires détectée ; et (iii) l'aimant de référence fixe détecté.

4. Procédé selon la revendication 1, dans lequel l'étape d'établissement comprend l'étape consistant à, exclusivement par palpation physique manuelle d'une zone d'intérêt, localiser un centre et une direction d'écoulement de l'unité de valve réglable.

5. Système de valve de drainage de liquide corporel programmable implantable, comprenant :
une valve de drainage de liquide corporel programmable implantable possédant une unité de valve réglable contenant une paire d'éléments magnétiques primaires servant à programmer la valve de drainage de liquide corporel programmable implantable à une valeur de paramètre souhaitée ;
une boîte à outils électronique universelle servant à indiquer et régler la valve de drainage de liquide corporel programmable implantable ; la boîte à outils électronique universelle comprenant :
un outil indicateur possédant un réseau de capteurs de détection de champ magnétique servant à : (i) détecter la paire d'éléments magnétiques primaires ; et (ii) déterminer la présence ou l'absence d'un aimant de référence fixe dans la valve de drainage de liquide corporel programmable implantable ;
un circuit servant à déterminer un centre de l'unité de valve réglable d'après la paire d'éléments magnétiques primaires détectée ; le circuit déterminant, exclusivement par une rétroaction électronique provenant de la boîte à outils, une orientation angulaire de la valve de drainage de liquide corporel programmable implantable lorsque l'aimant de référence fixe est présent ; le circuit générant sur un écran de la valve de drainage de liquide corporel programmable implantable les étapes permettant de déterminer, exclusivement par palpation physique manuelle, l'orientation angulaire de la valve de drainage de liquide corporel programmable implantable lorsque l'aimant de référence fixe est absent.

6. Système selon la revendication 5, dans lequel l'orientation angulaire est une direction d'écoulement de liquide à travers la valve de drainage de liquide corporel programmable implantable.

7. Système selon la revendication 5, dans lequel le circuit détermine le centre de l'unité de valve réglable :
en détectant, au moyen du réseau de capteurs de détection de champ magnétique, un motif de champ magnétique produit par chaque élément de la paire d'éléments magnétiques primaires ; et
en trouvant un point médian entre les éléments de la paire d'éléments magnétiques primaires détectée, représentant le centre de l'unité de valve réglable.

8. Système selon la revendication 7, dans lequel, lorsque la présence de l'aimant de référence fixe est détectée, le circuit détermine, exclusivement par une rétroaction électronique provenant de la boîte à outils, l'orientation angulaire de la valve de drainage de liquide corporel programmable implantable représentant une ligne de référence passant par : (i) une empreinte indiquant la direction d'écoulement de liquide à travers la valve de drainage de liquide corporel implantable ; (ii) le point médian trouvé entre les éléments de la paire d'éléments magnétiques primaires détectée ; et (iii) l'aimant de référence fixe détecté.
